# EUROPÄISCHE PATENTANMELDUNG

(11) **EP 1 394 170 A1**
(43) Veröffentlichungstag der Anmeldung: **03.03.2004**
(21) Anmeldenummer: 03015753.1
(22) Anmeldetag: 10.07.2003
(51) Int. Cl.: C07F 15/00, C07F 19/00, C07F 17/02

(54) **Verfahren zur Herstellung von cis-Alkenen und neue Katalysatoren hierfür**

(30) Priorität: 31.08.2002 DE 10240255
(71) Anmelder: Degussa AG, 40474 Düsseldorf (DE)
(72) Erfinder: Kirchhoff, Jochen, Dr., 59348 Lüdinghausen (DE); Fries, Guido, Dr., 45657 Recklinghausen (DE); Driessen-Hölscher, Birgit, Dr., 52074 Aachen (DE); Kalz, Willi, 52074 Aachen (DE); Nobis, Markus, Dr., 37603 Holzminden (DE)

(57) **Zusammenfassung**

Die Erfindung betrifft ein Verfahren zur Herstellung von *cis*-Alkenen durch selektive Hydrierung von konjugierten offenkettigen Diensystemen mit *in situ* dargestellten homogenen Übergangsmetallkatalysatoren. Die verwendeten aktiven Katalysatoren sind sehr leicht aus günstig vorhandenen Ausgangsverbindungen zugänglich.
Übergangsmetallkomplexe und Verfahren zu deren Herstellung.

## Beschreibung

Die vorliegende Erfindung richtet sich auf ein Verfahren zur selektiven homogenen katalytischen Hydrierung von konjugierten Doppelbindungssystemen zu cisoiden ungesättigten Verbindungen. Ebenfalls Gegenstand der Erfindung sind neue Katalysatoren hierfür, sowie ein Verfahren zu deren Herstellung.

Offenkettige cis-Alkene stellen wichtige Zwischenprodukte für die chemische Industrie dar. So führt die regio- und stereoselektive Hydrierung von Sorbinsäure beispielsweise zur einfach ungesättigten *cis*-3-Hexensäure, die für den Duftstoff- und Vitaminsektor von Intresse ist (S. Arctander, Perfume and Flavour Chemicals II, Montclair, USA, **1969**). Die aus der stereoselektiven Hydrierung von 2,4-ungesättigten Carbonsäureestern resultierenden *cis*-3-Carbonsäureester sind für die Pheromonsynthese von Bedeutung *(Mendeleev Commun.* **1994**, 4; *J. Org*. *Chem. USSR* **1991**, *27*, 824).

Regio- und stereoselektive Hydrierungen von offenkettigen konjugierten Diensystemen sind aus der Literatur bekannt.

*Alper* et al. untersuchten die Hydrierung konjugierter Diene unter Phasentransferbedingungen. Der als Katalysator verwendete Cobalt-Komplex [HCo(CN)₅³⁻] überführt ein 1,4-substituiertes Dien wie Sorbinsäure in ein Gemisch aus 75% *trans*-2-Hexensäure und 13 % *trans*-3-Hexensäure (*J. Org. Chem.* **1990,** *55*, 1854). *Cole-Hamilton* et al. berichten über eine regioselektive Hydrierung von Sorbinsäure, die 4-Hexensäure als Zielprodukt vor Augen hat (*J*. *Chem. Soc. Chem. Commun.* **1986,** 977; *J. Chem. Soc. Dalton Trans.* **1991,** 1929). Der verwendete *Wilkinson*-Katalysator war wenig aktiv, im Falle der Sorbinsäure findet man ausschließlich Hexansäure als Produkt. An einem heterogenen Palladiumkontakt gelang es *Kluson* et al. Sorbinsäuremethylester in trans-2-Hexensäuremethylester als Hauptprodukt zu überführen *(React. Kinet. Catal. Lett.* **1996,** *1*, 9). Ähnliche Arbeiten mit Katalysatoren auf Basis von Palladium, Platin, Rhodium und Ruthenium wurden zuvor von *Cerveny* et al. beschrieben *(Seifen,* Fette, *Öle, Wachse* **1990,** *116,* 549). Dabei konnte ebenfalls der 2-Hexensäureester als Zwischenprodukt isoliert werden.

11-Oxododeca-2,4-diensäuremethylester wurde unter Zugabe eines Chrom-Katalysators in das entsprechende Z-konfigurierte Alken überführt, in dem sich die ungesättigte Doppelbindung an Position 3 befindet *(Bioorg.Med.Chem.* **1996,** *4*, 389). *Corey* et al. beschreibt die Hydrierung von Dimethyl-(*E,E*)*-*(2-oxohepta-3,5-dienyl)phosphonat durch Vewendung eines Chromkatalysators *(J. Am. Chem. Soc.* **1984,** *106,* 3875). *Tucker* et al. untersuchten die selektive Hydrierung von 1,3-konjugierten Dienen zu *cis*-Monoenen mittels eines Chromcarbonyl-Komplexes *(J. Organomet. Chem.* **1985,** *279,* 49). *Vasil'ev* et al. beschreiben die selektive Hydrierung von konjugierten Dienen, die neben einer Esterfunktion auch eine Hydroxylgruppe tragen. So wird beispielsweise in einer Hydrierung aus Ethyl-12-hydroxydodeca-2,4-dienoat Ethyl-12-hydroxydodec-3-(*Z*)-enoat gebildet *(Izv. Akad. Nauk Ser. Khim.* **2000,** *49,* 1300). Andere Beispiele werden in *Zh. Org. Khim.* **1991,** *27*, 317 offenbart. Den Autoren gelingt es ebenfalls konjugierte Diene, die andere funktionelle Gruppen tragen wie zum Beispiel eine Ketal-Funktionalität *(Zh. Org. Khim.* **1991,** *27*, 966), eine zusätzliche Alken-Funktionalität *(Izv. Akad. Nauk Ser. Khim.* **1996,** *9*, 2350) oder eine Ether-Funktionalität *(Zh. Org. Khim.* **1991,** *27*, 966), in ein Z-konfiguriertes Alken zu überführen. Beispiele für die stereoselektive Hydrierung längerkettiger Carbonsäureester mit konjugierten Doppelbindungen zu den entsprechenden *cis*-3-Carbonsäureestern werden in *Mendeleev* *Commun.* **1994**, 4 gegeben. In allen Fällen wurde Hexacarbonylchrom oder Aren-tricarbonyl-Chromverbindungen als Katalysator verwendet.

Die stereoselektive Hydrierung von Sorbinsäureestern geht auf Arbeiten von *Frankel* et al. zurück *(Tetrahedron Lett.* **1968,** 1919; *J. Am. Chem. Soc.* **1968,** *90,* 2446). Die hierzu notwendige selektive *cis*-Addition des Wasserstoffs an das konjugierte Diensystem gelingt bei der katalytischen Hydrierung mit Chromcarbonylkomplexen. Die stereoselektive Hydrierung von Sorbinalkohol zum *cis*-3-Hexenol mit [Cr(CO)₆] oder [(Aren)Cr(CO)₃] als Katalysator wurde von *Furuhata* et al. beschrieben *(Agric. Biol. Chem.* **1982,** *46*, 1757). Dabei wurde eine Selektivität von etwa 95% erzielt, die Nebenprodukte sind hauptsächlich *trans*-3-Hexenol neben wenig 4-Hexenol und Hexanol.
Die stereoselektive Hydrierung von Sorbinsäure sowie Sorbinalkohol zu den entsprechenden cisoiden 3-Alkenverbindungen wurde auch von Drießen-Hölscher et al. beschrieben. Als Katalysatoren kamen definiert hergestellte Pentamethylcyclopentadienyl-Ruthenium-II-komplexe zum Einsatz *(Chem. Commun.,* **2000,** 217-218; *J. Prakt. Chem.* **2000,** 342, 348-354).

Aufgabe für die vorliegende Erfindung war die Angabe eines weiteren Verfahrens zur cisoiden Hydrierung von konjugierten Doppelbindungssystemen, dass unter ökonomischen und ökologischen Gesichtspunkten im technischen Maßstab besonders vorteilhaft einzusetzen ist. Insbesondere sollte das vorliegende Verfahren den prozesstechnischen und apparativen Aufwand möglichst gering gestalten und damit zur Kosteneffizienz beitragen helfen.

Diese und weitere sich aus dem Stand der Technik in naheliegender Weise ergebende Aufgaben werden durch ein Verfahren mit den Merkmalen des gegenständlichen Anspruchs 1 gelöst. Abhängige Unteransprüche richten sich auf bevorzugte Ausführungsformen des erfindungsgemäßen Verfahrens. Ansprüche 13 und 14 sind auf bevorzugte Übergangsmetallkomplexe des Rutheniums gerichtet, wohingegen Anspruch 15 bzw. 16 ein Verfahren zu deren Herstellung schützt.

Dadurch, dass man in einem Verfahren zur Herstellung von cis-Alkenen durch Hydrierung von konjugierten offenkettigen Diensystemen homogen lösliche Übergangsmetallkomplexe der Art (IIIa/b) 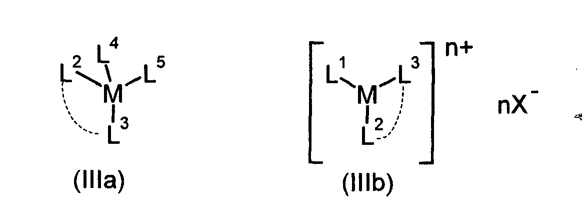 worin
in IIIa das Zentralatom ein Übergangsmetall der sechsten Gruppe des Periodensystems in der Oxidationsstufe ±0 ist, in IIIb das Zentralatom ein Übergangsmetall der achten Gruppe in der Oxidationsstufe +2, der neunten Gruppe in der Oxidationsstufe +1 oder der zehnten Gruppe des Periodensystems in der Oxidationsstufe +2 ist,
L¹ ist ein mehrzähniger neutraler oder anionischer Ligand,
L² und L³ bilden zusammen einen konjugierten offenkettigen Dien-Liganden,
L⁴ ist ein einzähniger, neutraler Ligand und
L⁵ ist ein mehrzähniger, neutraler Ligand,
X ist ein Anion und
n = 1,2 darstellt, einsetzt und die zur Hydrierung herangezogenen Übergangsmetallkomplexe dabei *in situ* herstellt, gelangt man völlig überraschend, dafür aber nicht minder vorteilhaft zur Lösung der gestellten Aufgabe. Im Stand der Technik sind bisher Verfahrensausführungen bei der Hydrierung vorgestellt worden, die alle samt die Generierung der einzusetzenden Katalysatoren in ihrer jeweils aktiven Form in einem von der Hydrierung getrennten vorgeschalteten Prozedere vorschlagen. Erstmals wird mit dem vorliegenden Verfahren die Möglichkeit aufgezeigt, diese vorgeschaltete Herstellung des aktiven Katalysators dadurch zu umgehen, dass wohlfeile Präkatalysatoren zur Reaktion herangezogen werden, die *in situ* in die aktiven Hydrierkatalysatoren überführt werden. Es ist insbesondere überraschend, dass diese *in situ*-Generierung des aktiven Katalysators völlig kompatibel zur eigentlichen Hydrierung ist und diese in keinster Weise negativ beeinflußt.

Vorteilhafterweise werden für die erfindungsgemäße Reaktion konjugierte Diene genommen, die einem Diensystem der allgemeinen Formel (II) 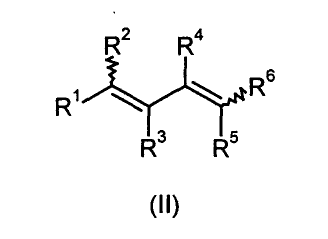 worin
R¹, R⁵ unabhängig voneinander stehen für H, (C₁-C₈)-Alkyl, (C₂-C₈)-Alkenyl, (C₂-C₈)-Alkinyl, (C₃-C₈)-Cycloalkyl, (C₆-C₁₈) -Aryl, (C₇-C₁₉)-Aralkyl, (C₃-C₁₈)-Heteroaryl, (C₄-C₁₉) -Heteroaralkyl, ((C₁-C₈)-Alkyl)₁₋₃-(C₃-C₈) -Cycloalkyl, ((C₁-C₈) -Alkyl)₁₋₃-(C₆-C₁₈)-Aryl, ((C₁-C₈)-Alkyl)₁₋₃-(C₃-C₁₈)-Heteroaryl,
R³, R⁴ unabhängig voneinander stehen für H, (C₁-C₈)-Alkyl, (C₂-C₈)-Alkenyl, (C₂-C₈)-Alkinyl, (C₃-C₈)-Cycloalkyl, (C₁-C₈)-Alkyl)₁₋ ₃-(C₃-C₈)-Cycloalkyl,
R², R⁶ unabhängig voneinander bedeuten H, HC(O)-, (C₁-C₈)-Acyl, COOR' mit R' = H oder (C₁-C₈)-Alkyl oder (C₇-C₁₉)-Aralkyl, (C₁-C₈)-Alkyl, (C₂-C₈)-Alkenyl, wobei diese Reste ggf. mit einer OH-, HC(O)-, (C₁-C₈)-Acyl-, -COOR' mit R' = H oder (C₁-C₈)-Alkylgruppe oder (C₇-C₁₉)-Aralkyl substituiert sein können, entsprechen.
Besonders bevorzugt ist in diesem Zusammenhang die Anwendung des gegenständlichen Verfahrens auf Verbindungen der Formel (II), bei denen in Konjugation zu dem Doppelbindungssystem eine C=O- oder eine C=N-Bindung oder eine C-O- oder C-N-Bindung vorhanden ist. Ganz besonders bevorzugt ist der Einsatz von Verbindungen gemäß Formel (II) ausgewählt aus der Gruppe der Sorbinsäure, Sorbinsäureester, Sorbinaldehyd oder Sorbinalkohol. Der Einsatz derartiger Verbindungen führt in sehr hohen Ausbeuten und sehr guten Selektivitäten zu Verbindungen der allgemeinen Formel (I) 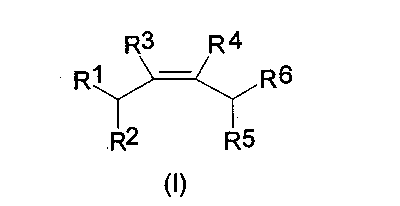 worin die Reste R¹ bis R⁶ die oben stehende Bedeutung annehmen können, welche wie schon angedeutet als Zwischenprodukte in vielerlei chemischen Synthesen Anwendung finden.

Als Zentralatome in den aktiven Übergangsmetallkomplexen, die als Katalysatoren für die erfindungsgemäße Hydrierung fungieren, sind insbesondere Metalle wie Chrom (Oxidationsstufe ±0)oder Molybdän (Oxidationsstufe ±0) im Falle von Komplexen der Art IIIa und im Falle von IIIb Eisen (Oxidationsstufe +2), Ruthenium (Oxidationsstufe +2), Cobalt (Oxidationsstufe +1) oder Rhodium (Oxidationsstufe +1) heranzuziehen. Ganz besonders bevorzugt ist der Einsatz von Ruthenium-Komplexen, in denen Ruthenium die Oxidationsstufe +II besitzt.

Die neutralen oder anionischen mehrzähnigen Liganden L¹ oder L⁵ in den Komplexen IIIa/b können prinzipiell vom Fachmann frei gewählt werden. Sie schirmen eine Halbsphäre der Übergangsmetallumgebung so ab, dass das Substrat sich nur von einer Seite dem Zentralatom so nähern kann, dass eine schematisierte Übergangsstruktur der folgenden Formel (IV) 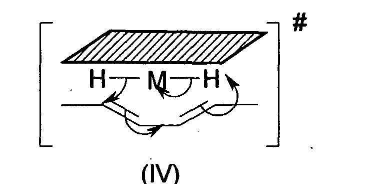 bevorzugt eingenommen wird (H.G. Niessen et al. *Magn. Reson. Chem.* **2000,** *38,* 747-750).

Besonders bevorzugt sind in diesem Zusammenhang Liganden, die mehr als vier Koordinationsstellen des eingesetzten Übergangsmetalls gleichzeitig besetzen können. Dieses sind u.a. Kronenether oder η^{5/6}-gebundene Liganden. Vorteilhaft ist, wenn in den Formeln IIIa/b L⁵ ein η⁶-gebundener Ligand und L¹ ein η⁵-gebundener Ligand vorliegt. Ganz besonderes bevorzugte η⁵-Liganden sind beispielsweise einfach oder mehrfach (C₁-C₈)-Alkyl- oder Silyl-substituierte Cyclopentadienyl- oder Indenylliganden oder Fluorenylliganden. Bevorzugt wird der Pentamethylcyclopentadienyl-Ligand. η⁶-Liganden sind Arene wie unsubstituiertes oder einfach ggf. mehrfach substituiertes Benzol, z. B. Benzol, Toluol, Mesitylen, Ethylbenzol, Anisol, (C₁-C₈) -Alkylbenzoat, Di- (C₁-C₈) -alkylphthaltate, -isophtahalate oder -terephthalate, 3-Carbo-C₁-C₈)-alkoxyanisole, Naphtaline oder Anthrazene.

Die Liganden L² und/oder L³ kann der Fachmann beliebig aus der Gruppe der konjugierten offenkettigen Diene auswählen. Er ist prinzipiell frei in deren Auswahl, solange diese kompatibel zur durchzuführenden Hydrierung sind. Bevorzugt wird man solche Diene als Liganden in den Komplexen vorliegen haben, die als Substrat zur Hydrierung eingesetzt werden. Dadurch wird eine Kontamination der Reaktionslösung mit cisoiden Nebenprodukten aus dem Katalysator vermieden. Besonders bevorzugte Liganden sind daher Sorbinalkohol, Sorbinaldehyd, Sorbinsäure oder Sorbinsäureester.

Die eingesetzten Katalysatoren tragen u. U. positive Ladungen. Um die Elektroneutralität zu wahren, wird dem aktiven Übergangsmetallkomplex ein nichtkoordinierendes Gegenion beigemengt. Der Fachmann ist unter der Prämisse frei in der Auswahl dieses Gegenions, als das es sich möglichst indifferent gegenüber der durchzuführenden Reaktion verhalten soll. Die Gegenionen können ausgewählt werden aus der Gruppe bestehend aus Cl⁻, Br⁻, I⁻, ClO₄⁻, BrO₄⁻, HSO₄⁻, ½ SO₄²⁻, NO₃⁻, PF₆⁻, PCl₆⁻, PBr₆⁻, AsF₆⁻, SbF₆⁻, CH₃SO₃⁻ (Mesylat), CF₃SO₃⁻ (Triflat), para-CH₃C₆H₄SO₃⁻ (Tosylat), (CF₃SO₂)₂N⁻ (Bis-trifluormethansulfonimid - BTA), oder ein Borat der Form BR'₄⁻ darstellen, wobei R' ein Halogen, insbesondere Fluor, einen (C₁-C₈)-Alkylrest, oder einen unsubstituierten oder einen einfach oder mehrfach mit Halogen, (C₁-C₈)-Alkyl, oder (C₁-C₈)-Haloalkyl substituierten (C₁-C₈)-Alkylrest oder einen (C₆-C₁₈)-Aryl, insbesondere Phenyl oder 3,5-Bis-(trifluormethyl)-phenyl bedeutet. Bevorzugt sind ClO₄⁻, PF₆⁻, PCl₆⁻, Mesylat, Triflat, Tosylat, BTA, BF₄⁻, BPh₄⁻, und BARF.

Wie schon angedeutet werden die Übergangsmetallkomplexe *in situ* gebildet. D. h., dass sogenannte Präkatalysatoren, welche leicht zugänglich sind, vor der Hydrierung in ihre aktive Form überführt werden, ohne dass eine Aufreinigung dieser Verbindungen vor ihrem Einsatz durch Isolierung erfolgen muss.

Die *in situ*-Katalysatoren stellt man im Falle von Chrom bzw. Molybdän als Übergangsmetallatom in einfacher Weise z. B. durch Umsetzung der Hexacarbonylkomplexe mit den jeweiligen Arenen zu den entsprechenden Aren-tricarbonyl-Komplexen her (C. Elschenbroich, A. Salzer, *Organometallchemie,* Teubner-Verlag, 2. Auflage, Stuttgart **1988**).

Im Falle von Ru als Übergangsmetallatom bietet es sich an, die *in situ*-Herstellung des Komplexes ausgehend von wohlfeilen dreiwertigen dimeren oder oligomeren Rutheniumhalogeniden, welche η⁵-Liganden aufweisen, durchzuführen. Durch Zugabe eines Reduktionsmittels, das befähigt ist, unter den gegebenen Bedingungen Ru-(III) in Ru-(II)-Verbindungen zu überführen, gelangt man zu monomeren Ru(II)-Komplexen, die die eigentliche aktive Spezies darstellen. Prinzipiell kann auch auf die Zugabe eines zusätzlichen Reduktionsmittel verzichtet werden. In diesem Falle erfolgt dann die Reduktion von Ru-(III) zur katalytisch aktiven Ru-(II)-Spezies mittels des unter Reaktionsbedingungen vorhandenen Wasserstoffs. Als Reduktionsmittel eignen sich neben Wasserstoff Metalle, wie beispielsweise Alkalimetall, Magnesium oder vorzugsweise Zink. Hierbei kann das Reduktionsmittel in metallischer Form vorliegen und/oder auf einem Träger aufgebracht sein. Weitere gängige Reduktionsmittel sind dem Fachmann geläufig *(Organikum,* Autorenkollektiv, Deutscher Verlag der Wissenschaften, 18. Auflage, Berlin **1990,** S. 288-295 und 430-439.). Z.B. kann [CpMe₅RuCl₂]₂ bzw. [CpMe₅RuCl₂]_{oligomer} durch Zugabe eines wie eben genannten Reduktionsmittels, durch Zugabe eines offenkettigen konjugierten Diens - speziell des zu hydrierenden Diens - und ggf. durch Zugabe eines Salzes der allgemeinen Form Mⁿ⁺X⁻ₙ, wobei M für ein Alkalimetall, Erdalkalimetall, Aluminum, Zink, Zinn oder Eisen und X wie oben beschrieben, steht, in den aktiven Übergangsmetallkomplex überführt werden. Im Hinblick auf z.B. entsprechende [(Indenyl)RuDien]- (s. Alvarez, Gimeno, *Organometallics,* **2001,** *20,* 3762-3771.) bzw. [(Fluorenyl)RuDien]-Komplexe (VI) und (VII) geht man vorteilhafter Weise von [(Indenyl)Ru(COD)Cl] bzw. [(Fluorenyl)Ru(COD)Cl] aus, die mit einem offenkettigen konjugierten Dien und mit einem Salz der allgemeinen Form Mⁿ⁺X⁻ ₙ, wobei M für ein Metall und X für ein Anion wie oben beschrieben steht, in Kontakt gebracht werden. Entsprechende Fluorenyl-Derivate können ausgehend von [(COD)RuCl₂]_{oligomer} durch Umsetzung mit Fluorenyl-Alkalimetall bzw. Tri-(C₁-C₈)-alkylsilyl-fluoren zum [(Fluorenyl)Ru(COD)Cl] erhalten werden. Im Anschluss an die in situ-Umwandlungen kann in erfindungsgemäßer Art und Weise sofort die Hydrierung erfolgen.

Ggf. kann dem Reaktionsgemisch auch eine Lewissäure zugesetzt werden. Beispiele geeigneter Lewis-Säuren sind unter anderem die entsprechenden Halogenide von Aluminium, Eisen, Zink, Zinn, Arsen oder Antimon oder halogenierte Borverbindungen wie zum Beispiel BF₃ oder BCl₃. Es können aber auch durchaus andere übliche Lewis-Säuren verwendet werden (J. March, *Advanced Organic Chemistry,* John Wiley & Sons. Inc., 3rd edition **1985,** S 227-229.).

Die Herstellung des *in situ* Katalysators erfolgt in der Regel in dem Lösungsmittel, in dem auch die Hydrierung des offenkettigen konjugierten Diens erfolgt. Besonders bevorzugt ist der Einsatz von einphasigen oder zweiphasigen Medien, wobei polare und/oder unpolare Lösemittel ausgewählt aus der Gruppe der Alkane, Aromaten, Ether, Polyether, Ester, Sulfoxide, Sulfone, Alkohole, Nitrile, Nitroalkane, Wasser oder aus der Gruppe der ionischen Flüssigkeiten verwendet werden. Es können aber auch durchaus Lösungsmittelgemische verwendet werden. Beispiele geeigneter Lösungsmittel je nach Wahl des Katalysatorsystems sind aromatische Lösungsmittel wie Benzol, Toluol, Ethylbenzol, Cumol, Mesitylen oder Xylole, Alkane oder Cyloalkane wie beispielsweise Hexan, Heptan, Decan, Cyclohexan oder Methylcyclohexan, Sulfoxide und Sulfolane wie Dimethylsulfoxid, Diisopropylsulfon, Sulfolan, 2-Methylsulfolan, 3-Methylsulfolan und 2-Methyl-4-butylsulfolan, Ether wie Diethylether, Methyl-tert.-butylether, Diisopropylether, Dibutylether, 1,2-Dimethoxethan, Tetrahydrofuran, Dioxan oder Anisol, Polyether wie beispielsweise Diethylenglykoldimethylether, Alkohole wie zum Beispiel Methanol, Ethanol, Isopropanol, Cyclohexanol, Methylcyclohexanol, 2-Ethylhexanol oder Diacetonalkohol, mehrwertige Alkohole wie Ethylenglykol oder Glycerin, Ester wie Ethylacetat, Butylacetat, Ethylpropionat, Ethylbutyrat oder Ethylenglykoldiacetat, Nitrile wie beispielsweise Acetonitril, oder Nitromethan. Es kann aber auch Wasser oder in ionischen Flüssigkeiten wie beispielsweise 1-Ethyl-3-methyl-imidazolium hexafluorophosphat oder 1-Ethyl-3-methyl-imidazolium tetrafluoroborat gearbeitet werden.

Der Wasserstoffdruck während der Reaktion liegt in der Regel in dem vom Fachmann zu wählenden Bereich. Er sollte so hoch liegen, dass die Reaktion möglichst rasch erfolgt. Auf der anderen Seite sollte die Hydrierung möglichst kostengünstig durchgeführt werden. Bevorzugt wird der Druck des Wasserstoffs während der Reaktion zwischen 1 bis 300 bar, vorzugsweise zwischen 5 und 60 bar liegen.

Die Temperatur, bei der die erfindungsgemäße Hydrierung durchgeführt wird, kann vom Fachmann beliebig gewählt werden. Hier sollte ebenfalls ein Ausgleich zwischen der Reaktionsgeschwindigkeit, die möglichst hoch sein sollte, und der Produktion von Nebenprodukten, die möglichst unterbunden werden sollte, erfolgen. Es hat sich gezeigt, dass Temperaturintervalle von 0°C bis 180°C, vorzugsweise 40°C bis 90°C, während der Reaktion zu bevorzugen sind.

Gegenstand der vorliegenden Anmeldung sind ebenfalls Übergangmetallkomplexe für die stereoselektive Hydrierung konjugierter Diene zu cisoiden Verbindungen aufweisend ein Ruthenium-(II)-Zentralatom, einen η⁵-gebundenen Liganden und einen zweizähnigen offenkettigen konjugierten Dien-Ligand sowie ggf. ein nicht koordinierendes Gegenion zur Herstellung der Elektroneutralität mit der Maßgabe, dass wenn der η⁵-gebundene Ligand Pentamethylcyclopentadienyl (Cp*) und das Dien Sorbinsäure ist, das Gegenion nicht das Triflat- bzw. das BARF-Anion sein darf. Im Rahmen dieser Vorgabe sind Übergangmetallkomplexe der Gruppe bevorzugt, bestehend aus Rutheniumverbindungen der allgemeinen Formel (V), (VI) oder (VII) 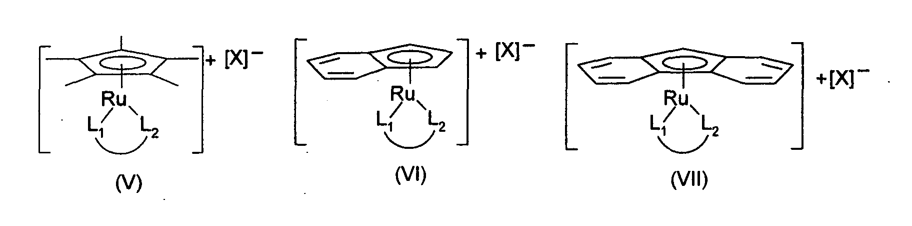 worin
L₁ und L₂ zusammen Sorbinsäureester, Sorbinaldehyd, Sorbinsäure oder Sorbinalkohol darstellen, und X⁻ ein Anion aus der Gruppe F⁻, Cl⁻, Br⁻, I⁻, ClO₄⁻, BrO4⁻, HSO4⁻, ½ SO₄²⁻, NO₃⁻, PF₆⁻, PCl₆⁻, PBr₆⁻, AsF₆⁻, SbF₆⁻, CH₃SO₃⁻ (Mesylat), CF₃SO₃⁻ (Triflat), para-CH₃C₆H₄SO₃⁻ (Tosylat), (CF₃SO₂)₂N⁻ (Bis-trifluormethansulfonimid, BTA) oder ein Borat der Form BR'₄⁻ darstellt, wobei R' ein Halogen, ein (C₁-C₈)-Alkylrest oder ein (C₆-C₁₈)-Arylrest sein kann.

Gegenstand der vorliegenden Erfindung ist auch ein Verfahren zur Herstellung von Verbindungen der allgemeinen Formel (V) durch Umsetzung von [CpMe₅RuCl₂]₂ bzw. [CpMe₅RuCl₂]_{oligomer} mit einem offenkettigen konjugierten Dien, einem Reduktionsmittel, das befähigt ist, unter den gegebenen Bedingungen Ru-(III) in Ru-(II)-Verbindungen zu überführen (s. o.) und wahlweise durch Zugabe eines Salzes der allgemeinen Form Mⁿ⁺X⁻ₙ, wobei M für ein Alkalimetall, Erdalkalimetall, Aluminum, Zink, Zinn oder Eisen, X für ein Anion wie oben bereits beschrieben steht.

Gegenstand der Erfindung ist ebenfalls ein Verfahren zur Herstellung von Verbindungen der allgemeinen Formel (VI) und (VII) durch Umsetzung von [(Indenyl)Ru(COD)Cl] bzw. [(Fluorenyl)Ru(COD)Cl] mit einem offenkettigen konjugierten Dien und einem Salz der allgemeinen Form Mⁿ⁺X⁻ₙ, wobei M für ein Metall und X für ein Anion wie oben beschrieben steht.

Die Lösungen der *in situ* hergestellten Katalysatoren können unter Schutzgas problemlos über Wochen gelagert werden. Eine weitere Reinigungsoperation wie beispielsweise eine Filtration oder Zentrifugation, um gegebenenfalls ausgefallene Feststoffanteile aus der Lösung zu entfernen, kann gegebenenfalls durchgeführt werden. Sie ist aber für den weiteren Verlauf des erfindungsgemäßen Verfahren von untergeordneter Rolle.

Als (C₁-C₈)-Alkyl sind anzusehen Methyl, Ethyl, n-Propyl, Isopropyl, n-Butyl, Isobutyl, sec-Butyl, tert-Butyl, Pentyl, Hexyl, Heptyl oder Octyl samt aller Bindungsisomeren. Diese können einfach oder mehrfach mit (C₁-C₈)-Alkoxy, (C₁-C₈)-Haloalkyl, OH, Halogen, NH₂, NO₂, SH, S-(C₁-C₈)-Alkyl substituiert sein.

Als (C₂-C₈)-Alkenyl ist mit Ausnahme von Methyl ein wie oben dargestellter (C₁-C₈)-Alkyl-Rest zu verstehen, der mindestens eine Doppelbindung aufweist.

Unter (C₂-C₈)-Alkinyl ist mit Ausnahme von Methyl ein wie oben dargestellter (C₁-C₈)-Alkyl-Rest zu verstehen, der mindestens eine Dreifachbindung aufweist.

Unter (C₁-C₈)-Acyl versteht man einen über eine -C=O-Funktion ans Molekül gebundenen (C₁-C₈)-Alkyl-Rest.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-atomhaltige Reste substituiert sein und/oder N-, O-, P-, S-atomhaltige Reste im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Ein (C₇-C₁₉)-Aralkylrest ist ein über einen (C₁-C₈)-Alkylrest an das Molekül gebundener (C₆-C₁₈)-Arylrest.

(C₁-C₈)-Alkoxy ist ein über ein Sauerstoffatom an das betrachtete Molekül gebundener (C₁-C₈)-Alkyl-Rest.

(C₁-C₈)-Alkoxycarbonyl ist ein über eine -OC(O)-Funktion an das betrachtete Molekül gebundener (C₁-C₈)-Alkyl-Rest. Dies gilt Synonym für die anderen Oxycarbonylreste.

(C₁-C₈)-Haloalkyl ist ein mit einem oder mehreren Halogenatomen substituierter (C₁-C₈)-Alkyl-Rest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Rest angesehen, wie 1-, 2-, 3-Furyl, wie 1-, 2-, 3-Pyrrolyl, 1-,2-,3-Thienyl, 2-, 3-, 4-Pyridyl, 2-, 3-, 4-, 5-, 6-, 7-Indolyl, 3-, 4-, 5-Pyrazolyl, 2-,4-, 5-Imidazolyl, Acridinyl, Chinolinyl, Phenanthridinyl, 2-, 4-, 5-, 6-Pyrimidinyl. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Unter einem (C₄-C₁₉)-Heteroaralkyl wird ein dem (C₇-C₁₉)-Aralkylrest entsprechendes heteroaromatisches System verstanden.

Halogen oder Hal bezeichnet Fluor, Chlor, Brom, Iod.

Die dargestellten Strukturen beziehen sich auf alle möglichen Diastereomere und bezüglich eines Diastereomers auf die darunter fallenden möglichen zwei (R)- und (S)-Enantiomere der in Frage stehenden Verbindung.

Die zitierten Dokumente gelten als von der Offenbarung mitumfasst.

Die folgenden Beispiele sollen das erfindungsgemäße Verfahren näher erläutern, nicht jedoch auf die speziell genannten Umstände einschränken.

### Beispiel 1: Herstellung der in situ Katalysatoren

### 1a) Darstellung von [CpMe₅RuCl₂]₂

Zu einer Lösung von RuCl₃ x H₂O (1.91g, 7.74mmol Ru) in 20 mL EtOH wird CpMe₅H (2.4g, 17.6mmol) gegeben. Der Reaktionsansatz wird 3h lang zum Rückfluß erhitzt, anschließend wird der entstandene dunkle Feststoff abfiltriert. Das Produkt wird dreimal mit Isopropanol und dreimal mit Ether gewaschen. Nach sehr gründlicher Trocknung im Hochvakuum fällt das Produkt als dunkelbraunes Pulver an.
Ausbeute: 1.68 g (70 %, 5.42 mmol)
¹H-NMR(300 MHz, CDCl₃): δ = 4.33 (m, C₅Me₅)

### Darstellung der in situ Katalysatoren

### 1b-1v) Darstellung von [CpMe₅Ru(Dien)]X-Verbindungen

### (1b-1r erfindungsgemäß)

### (1s-1v nicht erfindungsgemäß)

Zu einer Lösung von [CpMe₅RuCl₂]₂ (0.0245g, 0.08 mmol) in 10 mL Diethylether wird das entsprechende Dien (2.68 mmol), Zinkstaub (0.3g, 4.15 mmol) und 0,16 mmol eines Salzes gegeben Der Ansatz wird 2.5 h bei RT gerührt.

| **Katalysator** | **Dien** | **Salz** |
|---|---|---|
| 1b | Sorbinsäure | KOTf |
| 1c | Sorbinsäure | NaBF₄ |
| 1d | Sorbinsäure | NaBARF |
| 1e | Sorbinsäure | LiBF₄ |
| 1f | Sorbinsäure | LiPF₆ |
| 1g | Sorbinsäure | AgOTf |
| 1h | Sorbinsäure | Al(OTf)₃ |
| 1i | Sorbinsäure | Mg(OTf)₂ |
| 1j | Sorbinsäure | LiOTf |
| 1k | Sorbinsäure | LiBTA |
| 1l | Sorbinsäure | LiClO₄ |
| 1m | Sorbinalkohol | KPF₆ |
| 1n | Sorbinalkohol | LiClO₄ |
| 1o | Sorbinalkohol | NaBARF |
| 1p | Sorbinalkohol | NaBPh₄ |
| 1q | Sorbinalkohol | LiOTf |
| 1r | Sorbinalkohol | LiBTA |
| 1s | COD | NaBPh₄ |
| 1t | COD | LiBTA |
| 1u | COD | LiClO₄ |
| 1v | NBD | NaBF₄ |

### 1w-1z) Indenyl- bzw Fluorenyl-Ru-Sorbinsäure-Komplexe: (erfindungsgemäß)

0.08 mmol [(Indenyl)Ru(COD)Cl] bzw. [(Fluorenyl)Ru(COD)Cl] (hergestellt durch Umsetzung von Fluoren mit Kaliumhydrid und [(COD)RuCl₂]_{oligomer} in THF) werden in 10 mL Diethylether mit 2.68 mmol Sorbinsäure und 0,16 mmol eines Salzes gegeben Der Ansatz wird 2.5 h bei RT gerührt.

| **Katalysator** | **η**^{**5**}**-Ligand** | **Salz** |
|---|---|---|
| 1w | Indenyl | AgOCl₄ |
| 1x | Indenyl | AgOTf |
| 1y | Indenyl | NaBF₄ |
| 1z | Fluorenyl | AgOTf |

### Analytische Daten:

- [Cp*Ru(Sorbinsäure)]BF₄: (1c, 1e)
   ¹⁹F-NMR (282 MHz, CDCl₃) : -150.8 (BF₄⁻)
   SIMS: (m/z)⁺ = 348.9 ([Cp*Ru(sorb)]⁺], (m/z)⁻ = 70.0 (BF₄⁻)
- [Cp*Ru(COD)]BPh₄ (1s)
   SIMS: (m/z)⁺ = 343.1 ([Cp*RuCOD]⁺), (m/z)⁻=319.9(B(Ph)₄)⁻
- [Cp*Ru(COD)]BTA (1t)
   ¹H-NMR(300 MHz, CDCl₃): 1.51; (m, C₅Me₅), 2.0-4.0(m, COD).
   SIMS: (m/z)⁺:343.1([Cp*RuCOD]⁺),(m/z)=279.9(BTA⁻)
- [Cp*Ru(NBD)]BF₄ (1u)
   ¹H-NMR (300MHz, CDCl₃): 1.66 (m, C₅Me₅), 3.69(m, NBD), 4.13 (m, NBD).
   ¹⁹F-NMR (282 MHz, CDCl₃) : -151.0 (BF₄⁻)
- [(Indenyl)Ru(COD)]OTf (Präkatalysator für 1z)
   ¹H-NMR (300MHz, CDCl₃) : 2.10 (m, CH₂, COD), 3.72 (br s, CH, COD), 4.02 (br s, CH, COD), 5.57 (m, CH, C₉H₇), 7.35 (m, CH, C₉H₇)
   ¹⁹F-NMR (282 MHz, CDCl₃) : -78.37 (OTf⁻)
- [(Fluorenyl)Ru(COD)]OTf (Präkatalysator fur 1x)
   ¹⁹F-NMR (282 MHz, CDCl₃) : -78.54 (OTf⁻)

Sämtliche *in situ* hergestellten Katalysatoren können ohne weitere Reinigungsschritte der Hydrierung zugeführt werden.

### Hydrierversuche von Sorbinsäure mit Katalysatoren aus Beispiel 1 (erfindungsgemäB)

Sorbinsäure 12 mmol), das entsprechende Lösemittel und die Katalysator-Lösung werden in einen Stahlautoklaven(V2A-Stahl, 75 mL), welcher zuvor dreimal abwechselnd evakuiert und anschließend mit Argon geflutet wurde vorgelegt. Der Autoklav wird verschraubt und der gewünschte Wasserstoffdruck aufgepresst. Der Autoklav wird in ein vortemperiertes Ölbad gehängt und 30 min temperiert bevor der Rührer gestartet wird. Mit dem Einschalten des Rührers beginnt die Reaktionszeit. Die Reaktion wird abgebrochen, indem der Autoklav in einem Eisbad abgekühlt und anschließend durch vorsichtiges Öffnen des Nadelventils entspannt wird.

**Tabelle 1:**

| Hydrierung von Sorbinsäure in MTBE | | | |
|---|---|---|---|
| Kat. | Umsatz[%] | Selektivität [%] | |
| | | cis-3-Hexensäure | trans-3-Hexensäure |
| 1d | 71 | 97.1 | 2.9 |
| 1g | 100 | 82.6 | 14.4 |
| 1g *) | 26 *) | 98.1 *) | 1.9 *) |
| 1h | 71 | 90.0 | 10.0 |
| 1i | 14 | 98.1 | 1.9 |
| Reaktionsbedingungen: 0.08 mmol Kat., 12 mmol Sorbinsäure, 40 mL MTBE, 50 bar Wasserstoff, 70°C, 1 h Reaktionsdauer. - *) 20 bar, 50 °C, 3 h Reaktionsdauer. | | | |

**Tabelle 2:**

| Hydrierung von Sorbinsäure in Nitromethan | | | |
|---|---|---|---|
| Kat. | Umsatz[%] | Selektivität [%] | |
| | | cis-3-Hexensäure | trans-3-Hexensäure |
| 1b | 21 | 96.9 | 3.1 |
| 1c | 22 | 94.8 | 5.2 |
| 1d | 43 | 96.7 | 3.3 |
| 1e | 61 | 99.6 | 0.4 |
| 1f | 100 | 59.8 | 40.2 |
| 1f *) | 94 *) | 97.9 *) | 2.1 *) |
| 1k | 83 | 96.6 | 3.1 |
| 1l | 50 | 98.7 | 1.3 |
| 1s | 16 | 96.1 | 3.9 |
| 1t | 28 | 98.7 | 1.3 |
| 1u | 20 | 99.2 | 0.8 |
| Reaktionsbedingungen: 0.08 mmol Kat., 12 mmol Sorbinsäure, 40 mL Nitromethan, 50 bar Wasserstoff, 80°C, 4h Reaktionsdauer. - *) 2 h Reaktionsdauer. | | | |

### Hydrierversuche von Sorbinalkohol mit Katalysatoren aus Beispiel 1 (erfindungsgemäß)

**Tabelle 3:**

| Hydrierung von Sorbinalkohol in Nitromethan | | | |
|---|---|---|---|
| Kat. | Umsatz[%] | Selektivität [%] | |
| | ' | Cis-3-Hexenol | trans-3-Hexenol |
| 1m | 68 | 97.9 | 2.1 |
| 1m *) | 100 *) | 98.8 *) | 1.2 *) |
| 1n | 100 | 96.9 | 3.1 |
| 1o | 100 | 94.9 | 5.1 |
| 1p | 100 | 96.3 | 3.7 |
| 1q | 100 | 99.9 | 0.1 |
| 1r | 100 | 97.8 | 2.2 |
| Reaktionsbedingungen: 0.08 mmol Kat., 12 mmol Sorbinalkohol, 40 mL Nitromethan, 20 bar Wasserstoff, 60°C, 1h Reaktionsdauer. -*) 4 h Reaktionsdauer. | | | |

### Beispiel 2: In situ generierte Katalysatoren ausgehend von [Cp*Ru(COD)Cl] und Lewis-Säuren (erfindungsgemäß)

0.05 mmol [Cp*Ru(COD)Cl] werden in 10 ml THF gelöst. Nach Zugabe der entsprechenden Lewis-Säure (0.055 mol) wird die Mischung 60 Minuten bei Raumtemperatur gerührt. Anschließend wird die präformierte Katalysatorlösung und das jeweilige Lösemittel in den Autoklaven überführt.

### Hydrierversuche mit Katalysatoren aus Beispiel 2 und verschiedenen Lewis-Säuren (erfindungsgemäß)

**Tabelle 4:**

| Hydrierung von Sorbinsäure | | | | |
|---|---|---|---|---|
| Lewis-Säure | Lösemittel | Umsatz [%] | Selektivität [%] | |
| | | | ∑ Hydrierprodukte | cis-3-Hexensäure |
| Keine | THF | 0 | 0 | 0 |
| ZnCl₂ | THF | > 90 | > 98 | 10.5 |
| ZnCl₂ | CH₃NO₂ | < 5 | > 98 | 22.7 |
| ZnCl₂ | CH₃CN | < 5 | > 98 | > 98 |
| BF₃ | THF | < 5 | > 98 | 8.3 |
| SbCl₅ | THF | < 5 | > 98 | 9.9 |
| AlCl₃ | THF | < 5 | > 98 | 27.7 |
| Reaktionsbedingungen: 0.05 mmol Kat., 0,055 mmol Lewis-Säure, 10 mL Lösemittel, 9 mmol Sorbinsäure, 50 bar Wasserstoff, 80°C, 4 h Reaktionszeit. | | | | |

### Beispiel 3: Herstellung des in situ generierten Katalysators ausgehend von [CP*Ru(CH₃CN)₃]OTf (erfindungsgemäß)

Zu einer Lösung von [Cp*RuCl₂]₂ (0.0245g, 0.08 mmol) in 2 mL Acetonitril wird Zinkstaub (0.3g 4.15 mmol) und KOTf (0.09 mmol) gegeben. Der Ansatz wird 2.5 h bei RT gerührt. Anschließend kann die Katalysatorlösung so wie in Beispiel 1 beschrieben dem Hydrierversuch zugeführt werden.

**Tabelle 5:**

| Hydrierung von Sorbinsäure | | | |
|---|---|---|---|
| Salz | Umsatz [%] | Selektivität [%] | |
| | | cis-3-Hexensäure | Trans-3-Hexensäüre |
| KOTf | 17 | 98.7 | 1.3 |
| Reaktionsbedingungen: 0.08 mmol Kat. 12 mmol Sorbinsäure, 40 mL Nitromethan, 50 bar Wasserstoff, 80°C, 4 h Reaktionszeit, 0.09 mmol KOTf. | | | |

### Beispiel 4: In situ generierte Katalysatoren ausgehend von [(Indenyl)Ru(COD)Cl] (erfindungsgemäß)

0,08 mmol [(Indenyl)Ru(COD)Cl] wird in 20 mL Nitromethan gelöst und mit 0,1 mmol AgClO₄ versetzt. Nachdem die Mischung 30 Minuten bei 80°C gerührt worden ist, wird die Katalysatorlösung so wie in Beispiel 1 beschrieben dem Hydrierversuch zugeführt.

**Tabelle 6:**

| Hydrierung von Sorbinsäure | | | |
|---|---|---|---|
| Kat. | Umsatz [%] | Selektivität [%] | |
| | | Σ Hydrier-Produkte | Cis-3-Hexensäure |
| 1w | 5-10 | > 99 | 17.4 |
| Reaktionsbedingungen: 0.08 mmol Kat., 20 mmol Sorbinsäure, 20 mL Nitromethan, 50 bar Wasserstoff, 80 °C, 4 h Reaktionszeit | | | |

## Patentansprüche

1. Verfahren zur Herstellung von cis-Alkenen durch Hydrierung von konjugierten offenkettigen Diensystemen mit homogen löslichen Übergangsmetallkomplexen der Art (IIIa/b) 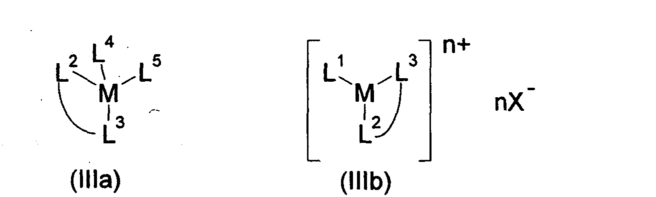 worin
in IIIa das Zentralatom ein Übergangsmetall der sechsten Gruppe des Periodensystems in der Oxidationsstufe ±0 ist, in IIIb das Zentralatom ein Übergangsmetall der achten Gruppe in der Oxidationsstufe +2, der neunten Gruppe in der Oxidationsstufe +1 oder der zehnten Gruppe des Periodensystems in der Oxidationsstufe +2 ist,
L¹ ist ein mehrzähniger neutraler oder anionischer Ligand,
L² und L³ bilden zusammen einen konjugierten offenkettigen Dien-Liganden,
L⁴ ist ein einzähniger, neutraler Ligand und
L⁵ ist ein mehrzähniger, neutraler Ligand,
X ist ein Anion und
n = 1, 2 darstellt, **dadurch gekennzeichnet, dass** die Übergangsmetallkomplexe *in situ* hergestellt zur Hydrierung herangezogen werden.

2. Verfahren nach Anspruch 1 **dadurch gekennzeichnet, dass** das Diensystem der allgemeinen Formel (II) 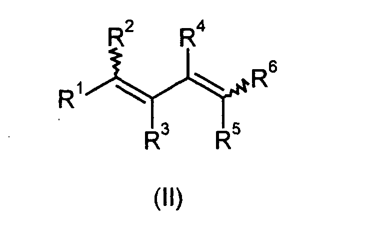 worin
R¹, R⁵ unabhängig voneinander stehen für H, (C₁-C₈)-Alkyl, (C₂-C₈)-Alkenyl, (C₂-C₈)-Alkinyl, (C₃-C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, ((C₁-C₈)-Alkyl)₁₋₃-(C₃-C₈)-Cycloalkyl, ((C₁-C₈)-Alkyl)₁₋₃- (C₆-C₁₈) -Aryl, ((C₁-C₈) -Alkyl)₁₋₃-(C₃-C₁₈) - Heteroaryl,
R³, R⁴ unabhängig voneinander stehen für H, (C₁-C₈)-Alkyl, (C₂-C₈)-Alkenyl, (C₂-C₈)-Alkinyl, (C₃-C₈)-Cycloalkyl, (C₁-C₈)-Alkyl)₁₋₃- (C₃-C₈) -Cycloalkyl,
R², R⁶ unabhängig voneinander bedeuten H, HC(O)-, (C₁-C₈)-Acyl, COOR' mit R' = H oder (C₁-C₈)-Alkyl oder (C₇-C₁₉)-Aralkyl, (C₁-C₈)-Alkyl, (C₂-C₈)-Alkenyl, wobei diese Reste ggf. mit einer OH-, HC(O)-, (C₁-C₈)-Acyl-, -COOR' mit R' = H oder (C₁-C₈)-Alkyl-gruppe oder (C₇-C₁₉)-Aralkyl substituiert sein können, entspricht.

3. Verfahren nach Anspruch 2, **dadurch gekennzeichnet, dass** die Verbindung II ausgewählt wird aus der Gruppe der Sorbinsäure, Sorbinsäureester, Sorbinaldehyd oder Sorbinalkohol.

4. Verfahren nach einem oder mehreren der vorhergehenden Ansprüche,
**dadurch gekennzeichnet, dass** das Zentralatom im Falle von IIIa Chrom oder Molybdän und im Falle von IIIb Eisen, Ruthenium, Cobalt oder Rhodium ist.

5. Verfahren nach Anspruch 1 und/oder 2, **dadurch gekennzeichnet, dass** L⁵ ein η⁶-gebundener Ligand ist und L¹ ein η⁵-gebundener Ligand ist.

6. Verfahren nach einem oder mehreren der vorhergehenden Ansprüche,
**dadurch gekennzeichnet, dass** L² und L³ einen zweizähnigen Liganden aus der Gruppe offenkettiger konjugierter Diene, wie Sorbinalkohol, Sorbinaldehyd, Sorbinsäure oder Sorbinsäureester, darstellen.

7. Verfahren nach einem oder mehreren der vorhergehenden Ansprüche,
**dadurch gekennzeichnet, dass** X ausgewählt ist aus der Gruppe bestehend aus Cl⁻, Br⁻, I⁻, ClO₄⁻, BrO₄⁻, HSO₄⁻, ½ SO₄²⁻, NO₃⁻, PF₆⁻, PCl₆⁻, PBr₆⁻, AsF₆⁻, SbF₆⁻, CH₃SO₃⁻ (Mesylat), CF₃SO₃⁻ (Triflat), para-CH₃C₆H₄SO₃⁻ (Tosylat), (CF₃SO₂)₂N⁻ (Bis-trifluormethansulfonimid - BTA), oder ein Borat der Form BR'₄⁻ darstellt, wobei R' ein Halogen, insbesondere Fluor, einen (C₁-C₈)-Alkylrest, oder einen unsubstituierten oder einen einfach oder mehrfach mit Halogen, (C₁-C₈)-Alkyl, oder (C₁-C₈)-Haloalkyl substituierten (C₁-C₈)-Alkylrest oder einen (C₆-C₁₈)-Aryl, insbesondere Phenyl oder 3,5-Bis-(trifluormethyl)-phenyl ist, bevorzugt BF₄⁻, BPh₄⁻, Tetrakis-[3,5-bis-(trifluormethyl)-phenyl]borat (BARF) ist.

8. Verfahren nach einem oder mehreren der vorhergehenden Ansprüche,
**dadurch gekennzeichnet, dass** der *in situ* Komplex im Falle von Ruthenium als Zentralatom ausgehend von [CpMe₅RuCl₂]₂ bzw. [CpMe₅RuCl₂]_{oligomer} durch Zugabe eines Reduktionsmittels, das befähigt ist, unter den gegebenen Bedingungen Ru-(III) in Ru-(II)-Verbindungen zu überführen, durch Zugabe eines offenkettigen konjugierten Diens und ggf. durch Zugabe eines Salzes der allgemeinen Form Mⁿ⁺X⁻ₙ, wobei M für ein Alkalimetall, Erdalkalimetall, Aluminum, Zink, Zinn oder Eisen und X gemäß Anspruch 7 beschrieben, steht, hergestellt wird.

9. Verfahren nach einem oder mehreren der vorhergehenden Ansprüche,
**dadurch gekennzeichnet, dass** die Hydrierung in Gegenwart einer Lewis-Säure durchgeführt wird.

10. Verfahren nach einem oder mehreren der vorhergehenden Ansprüche,
**dadurch gekennzeichnet, dass** dass die Umsetzung in einem einphasigen oder zweiphasigen Medium stattfindet , wobei polare und/oder unpolare Lösemittel ausgewählt aus der Gruppe der Alkane, Aromaten, Ether, Polyether, Ester, Sulfoxide, Sulfone, Alkohole, Nitrile, Nitroalkane, Wasser oder aus der Gruppe der ionischen Flüssigkeiten verwendet werden.

11. Verfahren nach einem oder mehreren der vorhergehenden Ansprüche,
**dadurch gekennzeichnet, dass** der Wasserstoffdruck während der Reaktion bei 1 bis 300 bar, vorzugsweise bei 5 bis 60 bar liegt.

12. Verfahren nach einem oder mehreren der vorhergehenden Ansprüche,
**dadurch gekennzeichnet, dass** die Temperatur während der Reaktion 0°C bis 180°C, vorzugsweise 40°C bis 90°C beträgt.

13. Übergangsmetallkomplexe für die stereoselektive Hydrierung konjugierter Diene zu cisoiden Verbindungen aufweisend ein Ruthenium-(II)-Zentralatom, einen η⁵-gebundenen Liganden und einen zweizähnigen offenkettigen konjugierten Dien-Ligand sowie ggf. ein nichtkoordinierendes Gegenion zur Herstellung der Elektroneutralität mit der Maßgabe, dass wenn der η⁵-gebundene Liganden Pentamethylcyclopentadienyl (Cp*) und das Dien Sorbinsäure ist, das Gegenion nicht das Triflat- bzw. das BARF-Anion sein darf.

14. Übergangmetallkomplexe nach Anspruch 13 ausgewählt aus der Gruppe bestehend aus Rutheniumverbindungen der allgemeinen Formel (V), (VI) oder (VII) 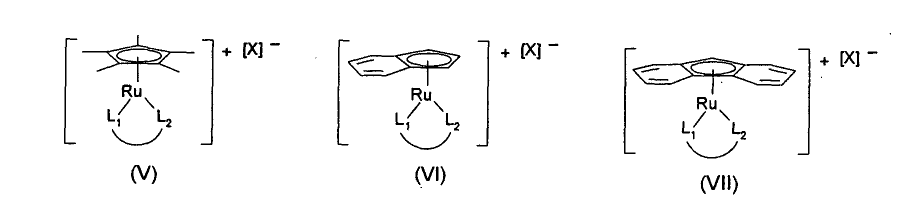 worin
L₁ und L₂ zusammen Sorbinsäureester, Sorbinaldehyd, Sorbinsäure oder Sorbinalkohol darstellen, und X⁻ ein Anion aus der Gruppe F⁻, Cl⁻, Br⁻, I⁻, ClO₄⁻, BrO₄⁻, HSO₄⁻, ½ SO₄²⁻, NO₃⁻, PF₆⁻, PCl₆⁻, PBr₆⁻, AsF₆⁻, SbF₆⁻, CH₃SO₃⁻ (Mesylat), CF₃SO₃⁻ (Triflat), para-CH₃C₆H₄SO₃⁻ (Tosylat), (CF₃SO₂)₂N⁻ (Bis-trifluormethansulfonimid, BTA) oder ein Borat der Form BR'₄⁻ darstellt, wobei R' ein Halogen, ein Alkylrest, ein unsubstituierter Arylrest, oder ein einfach oder mehrfach halogen-, alkyl- oder perfluoralkyl-substiuierter Arylrest ist.

15. Verfahren zur Herstellung von Verbindungen der allgemeinen Formel (V) durch Umsetzung von [CpMe₅RuCl₂]₂ bzw. [CpMe₅RuCl₂]_{oligomer}) mit einem offenkettigen konjugierten Dien, einem Reduktionsmittel, das befähigt ist, unter den gegebenen Bedingungen Ru-(III) in Ru-(II)-Verbindungen zu überführen, und ggf. durch Zugabe eines Salzes der allgemeinen Form Mⁿ⁺X⁻ₙ, wobei M für ein Alkalimetall, Erdalkalimetall, Aluminum, Zink, Zinn oder Eisen, X für ein Anion wie in Anspruch 7 bereits beschrieben steht.

16. Verfahren zur Herstellung von Verbindungen der allgemeinen Formel (VI) und (VII) durch Umsetzung von [(Indenyl)Ru(COD)Cl] bzw. [(Fluorenyl) Ru (COD) Cl] mit einem offenkettigen konjugierten Dien und einem Salz der allgemeinen Form Mⁿ⁺X⁻ₙ, wobei M für ein Metall und X für ein Anion wie in Anspruch 7 bereits beschrieben steht.
